# EUROPEAN PATENT APPLICATION

(11) **EP 3 508 135 A1**
(43) Date of publication of application: **10.07.2019**
(21) Application number: 18150313.7
(22) Date of filing: 04.01.2018
(51) Int. Cl.: A61B 8/12, A61B 8/08, A61B 8/00

(54) **CALIBRATION BLOCK, CALIBRATION SYSTEM AND CALIBRATION METHOD**

(71) Applicant: Koninklijke Philips N.V., 5656 AE Eindhoven (NL)
(72) Inventor: VAN ROOIJ, Johannes Antonius, 5656 AE Eindhoven (NL); HAUTVAST, Guillaume Leopold Theodorus Frederik, 5656 AE Eindhoven (NL); GERAATS, Jacobus Sigbertus Marie, 5656 AE Eindhoven (NL)
(74) Representative: de Haan, Poul Erik

(57) **Abstract**

The invention relates to a calibration block for determining a transformation between an ultrasound (US) image and a tracking coordinate system, the calibration block (10) comprising a mounting part (20) for mounting an ultrasound probe (2) to the calibration block (10) in a locked position, and a guidance pathways defining part (30) for defining a plurality of coplanar guidance pathways (32), wherein the calibration block (10) is configured to receive the ultrasound probe (2) in the locked position so that the plurality of guidance pathways (32) is aligned with an imaging plane of the ultrasound probe (2). The invention further relates to a corresponding method and computer program. The invention provides provide an improved calibration apparatus, calibration system and corresponding calibration method for determining a transformation between a US image and a tracking coordinate system.

## Description

### FIELD OF THE INVENTION

The invention relates to a calibration block and a calibration system for determining a transformation between an ultrasound (US) image and a tracking coordinate system. The invention further relates to a corresponding method and computer program. It finds particular application in clinical applications such as transrectal ultrasonography (TRUS) for brachytherapy, wherein the invention is of course not limited to this field.

### BACKGROUND OF THE INVENTION

Determining an accurate calibration or registration between anatomical locations visible in an US image and tracked probes located in the region imaged by the US probe is challenging. Sound reflections of a 3D volume are imaged, which has an extension normal to a 2D imaging plane of the US probe. Tracked probes having reflective parts will thus be visible in the US image even if they are out of the 2D imaging plane, e.g. at the edge of the 3D imaged volume.

Currently known calibration processes to determine an accurate calibration are highly operator dependent and time consuming to ensure the position of the tracked probes be determined while on the 2D imaging plane, i.e. being "on plane".

### SUMMARY OF THE INVENTION

It has thus been an object of the present invention, to provide an improved calibration apparatus, calibration system and corresponding calibration method for determining a transformation between a US image and a tracking coordinate system.

In a first aspect, a calibration block for determining a calibration between a US image and a tracking coordinate system is provided. The calibration block comprises
a mounting part for mounting a US probe to the calibration block in a locked position, and
a guidance pathways defining part for defining a plurality of coplanar guidance pathways,

wherein the calibration block is configured to receive the US probe in the locked position so that the plurality of guidance pathways is aligned with an imaging plane of the US probe.

The calibration block can thus be mounted on a US probe, wherein the user carrying out the calibration does not have to bother establishing the in or out of plane position of a device being tracked in the tracking coordinate system. Since the guidance pathways are coplanar and in line with the imaging plane of the ultrasound probe, the position of any tracked device received within the defined guidance pathways is advantageously automatically aligned with the US imaging plane.

Further, since the position of the US probe, at which the US probe is mounted within the mounting part, is a locked position, the position is fixed and thus accurately known. In this context, a locked position shall refer to a predetermined, repeatedly achievable position of the US probe. Expressed differently, the position of the US probe is precisely defined by the mounting part in several degrees of freedom (DOF).

In an embodiment, the calibration block further comprises an acoustic slot extending from the mounting part through the guidance pathways defining part, wherein the acoustic slot intersects the guidance pathways.

The acoustic slot can be a slot carved out of the rest of the calibration block and/or can comprise acoustically transparent medium. The acoustic slot preferentially corresponds to or comprises the acoustic scan volume scanned by the US probe in the locked position and thus preferentially begins adjacent to the US probe in the locked position. Since the guidance pathways intersect with the acoustic slot, tracked devices being received within the guidance pathways will automatically also pass the acoustic slot, i.e. the US scan volume of the US probe.

In an embodiment the calibration block comprises a first and a second acoustic slot, each extending from the mounting part through the guidance pathways defining part, wherein the first and second acoustic slot extend in a different direction, respectively.

Advantageously, the calibration block can thus be employed in calibrating a biplanar US probe, i.e. a probe comprising two imaging probes for imaging two different planes, for instance two planes being substantially perpendicular to each other. Thus, preferentially, the second acoustic slot is arranged in a transverse imaging plane, i.e. substantially perpendicular to the plane in which the guidance pathways extend. In contrast, the first acoustic slot is preferentially arranged in the sagittal imaging plane, i.e. in the plane in which the guidance pathways extend. The two acoustic slots can intersect or be separated from each other.

In an embodiment the guidance pathways defining part defines three coplanar guidance pathways. In this embodiment, three guidance pathways are preferentially arranged in parallel with respective different distances from the US probe. It should be noted that in other embodiments of course also more than three coplanar guidance pathways are feasible.

In an embodiment the mounting part is configured to secure the ultrasound probe in six degrees of freedom. Preferentially, the US probe can thus neither be translated nor rotated within the locked position. Thus, the calibration can be carried out accurately. Preferably, at least one of the degrees of freedom of the US probe is blocked by an elastical deformation of at least a part of the mounting part.

In an embodiment, the calibration block comprises a fixation component for securing the ultrasound probe in the locked position. Preferably, the fixation component comprises at least one screw. However, also other fixation components are of course contemplated.

Preferentially, the fixation component is arranged for pulling the ultrasound probe against the calibration block, more particular against a surface comprising the acoustical window leading to the guidance pathways. Thus, the fixation component assures the positioning of the ultrasound probe with respect to the scanning direction of the US probe.

In an embodiment, the calibration block comprises a plastics material, in particular polytetrafluoroethylene (PTFE).

A plastics material comprises a sufficient elasticity to assure for the ultrasound probe to be elastically fixed within the locked position. Yet, plastics material and in particular PTFE are of course only examples for suitable materials and other materials of the calibration block are contemplated.

It is important that the calibration block comprise a material, which does not interact with or interfere with the tracking system, for instance an electromagnetic (EM) tracking system and the corresponding electromagnetic field.

Further, it should be noted that the calibration block can of course be made of a single piece or multiple parts, such as a separate mounting part and separate guidance pathways defining part, can be attached together.

In a further aspect, a calibration system for determining a transformation between an ultrasound (US) image and a tracking coordinate system is provided. The calibration system comprises:
a calibration block according to an aspect of the present invention, and
a guidance component for being inserted into at least one of the guidance pathways, wherein the guidance component is configured to guide a feature visible in an ultrasound image and a tracked feature along the guidance pathway.

Since the guidance component is configured to guide both the feature visible in the ultrasound image and the tracked feature along the guidance pathway, and the since the guidance way is aligned with the ultrasound imaging plane, a transformation between the US image and the tracking coordinate system can be determined.

In an embodiment, the calibration system further comprises a processing unit. The processing unit is configured to:
record multiple corresponding positions of the feature visible in the US image and the tracked feature along the guidance pathway, and to
determine a transformation between the US image and the tracking coordinate system based on the recorded multiple corresponding positions.

The processing unit can be integrated, for instance, in the processing component of the US scanner or be provided in the form of a separate processing unit. Further, one, more or all of the features implemented in the processing unit can be distributed over multiple processing units, even including servers accessed via the Internet.

Since the plurality of guidance pathways are all aligned with the imaging plane of the ultrasound probe, every position along the guidance pathway of the guidance component including visible features of both ultrasound and tracked system can be recorded, wherein all such positions have the ultrasound image feature lying in the plane of the ultrasound probe imaging plane. Accordingly, the difficulty of aligning the ultrasound feature with the ultrasound imaging plane is avoided by using the calibration block according to the invention.

Preferentially, the ultrasound positions are recorded at a same time as the tracked features are recorded, so that several two corresponding point clouds of subsequent acquisitions are constructed in the ultrasound and tracking coordinate system. Preferentially, this recording is carried out through all of the guidance pathways using multiple recording positions in each of the guidance pathways. The recoding can be done, for instance, by marking a respectively visible ultrasound feature on a screen or the like and by concurrent acquisition of the position of the tracked feature. The two point clouds can then be transformed into each other by known methods, for instance, through singular value decomposition methods that establish a transformation matrix between both coordinate spaces.

With this transformation, e.g. the transformation matrix, the US images are projected into the tracking coordinate system in which also the features are tracked. In an embodiment, a relative position of the feature visible in the ultrasound image and the tracked feature is fixed.

Since the relative position of the feature visible in the ultrasound image and the tracked feature is fixed, a determined position of a feature visible in the ultrasound image can be related to the tracked feature by simply adding the offset corresponding to the fixed relative separation. Preferentially, the extension direction of the guidance pathways is defined as extending along one axis and the relative position of the feature visible in the ultrasound image and the tracked feature only extends in this extension direction of the guidance pathways. Further preferably, both features are arranged at the center of the guidance pathways. This facilitates calculation even further.

In an embodiment, the system further comprises a calibration needle comprising a tracked sensor component, wherein the guidance component is configured to receive the calibration needle therein.

The calibration needle preferentially comprises the sensor component on a tip thereof. Thus, the tracked feature, i.e. corresponding to the tracked sensor component, corresponds to the most distally arranged part of the needle. The calibration needle preferentially resembles the needles used in clinical practice, while the calibration needles can of course also be different. Since the calibration needle can be received within the guidance component, both needle and the tracked sensor component comprised therein can be positioned along the coplanar guidance pathways lying within the ultrasound image plane easily.

In an embodiment, the guidance component comprises a guidance element and a trocar shaft to be mounted within the guidance element, wherein a tip of the trocar shaft corresponds to the feature visible in an ultrasound image.

Preferentially, the trocar shaft including trocar tip can be mounted within the guidance element, wherein a calibration needle can be received inside the trocar shaft. Preferentially, an axis of the trocar shaft corresponds to a central axis of the guidance element, wherein the calibration needle further preferentially can be received at the same axis with the trocar shaft. While a tip of a trocar shaft has an advantageous visibility in ultrasound images, the shape is of course not limited to trocar tips and also further arrangements, which generate good contrast in ultrasound images, are likewise contemplated.

In an embodiment, the guidance element comprises an acoustical aperture perpendicular to the direction along the guidance pathway, wherein the tip of the trocar shaft is acoustically visible through the acoustical aperture.

An acoustical aperture can of course be a simple opening or hole, and can alternatively include acoustically transparent material provided in the acoustical aperture. The direction of the acoustical aperture being perpendicular to the direction along the guidance pathway preferentially corresponds to the extension direction of the acoustic slot of the calibration block, when the guidance element is received within the guidance pathway. The tip of the trocar shaft is thus, since its reflections are not deteriorated by the guidance element itself, accurately visible in the obtained ultrasound image.

In an embodiment, the trocar shaft comprises a needle channel for receiving a calibration needle therein, wherein an end of the needle channel is located at a determinable position with respect to the tip of the trocar shaft.

Preferentially, the end of the needle channel is arranged at a central axis of the trocar shaft, wherein a tracking sensor mounted in the calibration needle is thus provided at a known position from the trocar tip. Further preferentially, both the trocar shaft and the calibration needle are thus arranged at a known position, preferentially in the center of the guidance element.

In an embodiment both the guidance pathways and the guidance component have a cylindrical shape so that the guidance component is rotatable when the guidance component is received within one of the guidance pathways.

By rotating the guidance component within the guidance pathway, a misalignment of the ultrasound probe can be determined in case the feature visible in the ultrasound image is not completely rotationally symmetrical. In this example, deviations from a constant position can be identified in the ultrasound image. Further, both the guidance pathways and guidance component being formed cylindrically allows for an easier positioning of the guidance component within the guidance pathways. However, the shape is of course not limited as a cylindrical shape and also other guidance components, which are not rotatable, are contemplated.

In an embodiment, the tracked feature is an electromagnetically (EM) tracked feature. Accordingly, the tracking coordinate system is in this embodiment preferentially a coordinate system of an electromagnetical (EM) tracking system. EM tracking systems are known to provide an accurate and reproducible tracking result unless the quality of the EM field is insufficient. Nevertheless, the invention is of course not limited to EM tracking system and also further tracking systems, such as optical tracking systems, are contemplated.

In an embodiment, the system further comprises an end stop component. The end stop component is configured to be inserted into one of the guidance pathways for defining a limit stop of the guidance component along the guidance pathway.

The end stop component can for instance correspond to a plug to be inserted opposite to where the guidance component is inserted into the guidance pathway. In a preferred embodiment, the limit stop is thus configured that the guidance component can rest at a predetermined reproducible position within the guidance pathway. Further preferably, in case the calibration block comprises the first and second acoustic lot, wherein the second acoustic slot corresponds to a transverse imaging plane, the guidance component can be arranged precisely within the transversal imaging plane through the end stop component. The end stop component can be attached to the guidance block by fixating means, e.g. screws, and different sizes for defining different limit stops within the guidance pathways can be provided.

According to a further aspect, a guidance component for use with a calibration block for determining a transformation between an US image and a tracking coordinate system according to an aspect of the present invention is provided. The calibration block comprises a guidance pathways defining part for defining a plurality of coplanar guidance pathways for being inserted into at least one of the guidance pathways, the guidance component being configured to guide a feature visible in an ultrasound image and a tracked feature along the guidance pathway.

According to a further aspect, a calibration method for determining a transformation between an ultrasound (US) image and a tracking coordinate system using a calibration system according to an aspect of the invention is provided. The method comprises:
mounting an ultrasound probe to the mounting part of the calibration block,
inserting a guidance component into a guidance pathway, wherein the guidance component is configured to guide a feature visible in an ultrasound image and a tracked feature along the guidance pathway,
recording multiple corresponding positions of the feature visible in the ultrasound image and the tracked feature at the same position of the guidance component, and
determining a transformation between the ultrasound image and the tracking coordinate system based on the recorded multiple corresponding positions.

According to a further aspect, a computer program for determining a transformation between an ultrasound (US) image and a tracking coordinate system is provided. The computer program comprises program code means for causing a processing unit to carry out the calibration method as defined in claim 14, when the computer program is run on a processing unit of a calibration system as defined in claim 6.

It shall be understood that the calibration block of claim 1, the calibration system of claim 6, the calibration method of claim 14, and the computer program of claim 15, have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Figs. 1A-1C schematically and exemplarily illustrate the ultrasound image dimensions,
Figs. 2A-2C schematically and exemplarily illustrate a needle visualization in a 2D ultrasound image,
Fig. 3 schematically and exemplarily illustrates an example of a calibration block according to the invention,
Fig. 4 schematically and exemplarily illustrates an example of an ultrasound probe,
Fig. 5 schematically and exemplarily illustrates the mounting of an ultrasound probe to a calibration block,
Fig. 6 schematically and exemplarily illustrates a calibration needle,
Fig. 7 schematically and exemplarily illustrates a calibration needle inside a trocar shaft,
Fig. 8 schematically and exemplarily illustrates a guidance component comprising a trocar shaft,
Figs. 9A and 9B schematically and exemplarily illustrate a calibration system in cross section,
Fig. 10 schematically and exemplarily illustrates an ultrasound image acquired with the calibration system,
Fig. 11 schematically and exemplarily illustrates recorded calibration locations,
Fig. 12 schematically and exemplarily illustrates a flowchart of a calibration method,
Fig. 13A and 13B schematically and exemplarily illustrate an end stop component and a further embodiment of the calibration system, and
Fig. 14 schematically and exemplarily illustrates an example with a second acoustic slot.

### DETAILED DESCRIPTION OF EMBODIMENTS

In several clinical procedures, ultrasound (US) systems are developed to supplement or replace other imaging techniques, such as X-ray, computed tomography (CT) and magnetic resonance imaging (MRI), since the established or known techniques are expensive. Exemplarily, a clinical procedure that can benefit from US systems due its favorable costs is prostate brachytherapy. In brachytherapy, a radiation dose is delivered using catheters or needles as pathways to deliver radiation sources into the region to be treated of the patient. How much radiation dose a part of the body receives is crucial for success of the brachytherapy procedure and it depends on distance and dwell time of the radiation source with respect to that part of the body. In order to determine the radiation dose intensity, a distance and a position of the anatomy and the catheter or needle must thus be known in a coordinate system with the required accuracy. Just to give an example, in the context of brachytherapy, an accuracy of 2mm within a confidence interval of 95% is generally required, which is - for respective different reasons concerning both the anatomy and the catheter - challenging to achieve based on US.

In order to determine the location of the anatomy, one or more MRI or other images, for instance in DICOM format, are overlaid with the live US image to compensate the lack of resolution of cancerous tissue against healthy tissue in ultrasound. By using the information of the additional images during the segmentation of the anatomy in the US image, e.g. the lesion such as the cancerous tissue and other prostate part positions are known in this US image. Thereby, only one MRI or other image, which is more expensive than a US acquisition, is needed in advance. During the treatment session, it is then sufficient to acquire live US images.

A further difficulty is the location of the catheter or needle. The resolution of 3D images is not high enough and 2D images suffer from a large in or out of plane detection error. Operator dependencies on how accurate the localization can be carried out, were shown to commonly exceed 3mm, which is higher than the required accuracy, for instance by Racine et al. in their article "Characterization of Electromagnetic versus Manual 3DUS-Based Catheter Tip Localization Errors in High Dose Rate Brachytherapy Procedures" Brachytherapy 15:S194:2016.

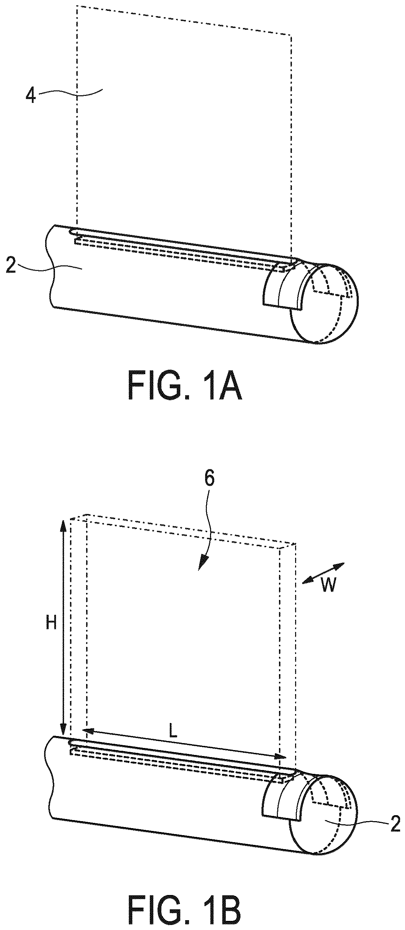

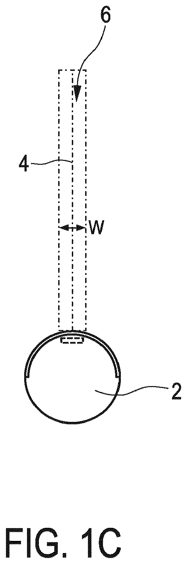

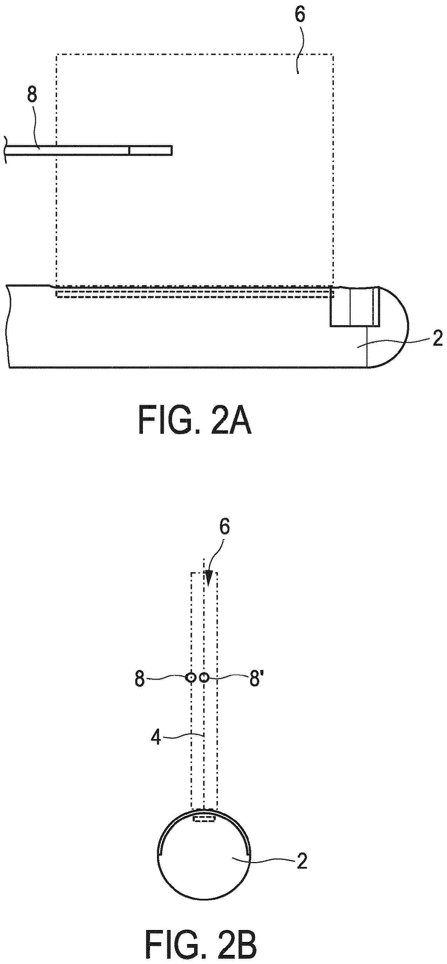

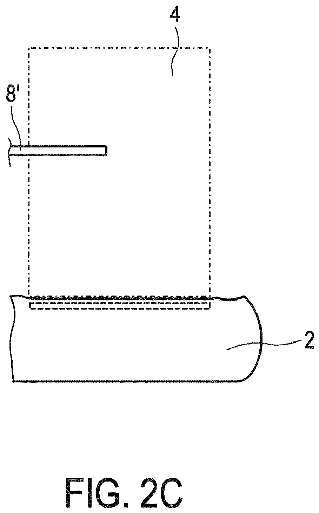

The difficulties of in or out of plane detection of features in US images will be described with reference to Figs. 1 and 2 below.

To improve the accuracy of the needle or feature identification in US images, a tracking system, such as an electromagnetic (EM) tracking system, which tracks the position of the US image and the location of the one or more catheters, is used. By having the position information of both the US image, i.e. the anatomies visible in the US image, and the catheters available in the coordinate system of the tracking system, e.g. the EM tracking system, distances between catheter and anatomy parts can be calculated with the required accuracy.

In order to have the positions of the electromagnetically tracked probes and the anatomies in a single coordinate system, a transformation between positions of the US image and the positions of the EM tracked devices has to be established. Such transformation, which defines a mapping between both coordinate systems, can, for instance, be employed using a transformation matrix. Expressed differently, the transformation, e.g. transformation matrix, can be used to transform the US images to the correct position in the EM tracking coordinate system. Since the EM tracked devices are already located within this coordinate system, the tracking coordinate system, anatomies and EM tracked devices or features will be in the same coordinate system.

During the calibration, a position of a feature which is visible in both the US image and the EM tracking system and of which the location is known in these separate coordinate systems is locked or recorded at the same time. By repeating this for several positions, two point clouds can be established in each of the two coordinate systems, which can then be used to establish a transformation matrix that transforms one point cloud onto the other with the smallest possible overlap error. For example, singular value decomposition or other methods can be used for establishing the transformation matrix.

The accuracy of the transformation matrix highly depends on the accuracy of the marked and locked positions of the features in both systems. For example, considering the out of plane effect will be described with reference to Fig. 1 and 2, it is difficult to know based on a US image, whether the visible feature is indeed in plane or not.

In Fig. 1A, a 2D imaging plane 4 of an ultrasound probe 2 is illustrated. The 2D plane 4 has no extension normal to the plane. In fact, a 3D volume 6, as illustrated in Fig. 1B, is used by the algorithm in the US system to construct the 2D image. As can be seen in the cross-sectional view in Fig. 1C, the 3D volume 6 has an extension w normal to the 2D imaging plane. Just to give exemplary numbers, the width w can amount to up to ten percent of a height H and a length L. More precisely, if for instance the 2D US image has an extension of, for instance, 64 to 65mm, a width of the 3D volume 6 can be, for instance, 6mm.

Fig. 2A to 2C schematically and exemplarily illustrate the effect of a needle 8 present in the 3D volume 6. Even if, as can be seen in Fig. 2B, needle 8 is displaced relative to 2D plane 4, for instance, to the left as illustrated in Fig. 2B, a projected position 8' of needle 8 is visible in the US image, as can be seen in Fig. 2C. Expressed differently, needle 8 will be shown as if it were in 2D plane 4, even though it is displaced several mm normal to the imaging plane. This effect makes it hard to distinguish between needle positions 8 and 8', as illustrated in Fig. 2B, and causes an operator dependent calibration in the accuracy when calibrating with, for instance, needle tips presenting no further distinguishable features.

In order to achieve the required accuracy, previously known calibration methods are time-consuming and are operator dependant. To increase the accuracy, many data points are needed and multiple determination are necessary to know whether the device is in plane or not by repeating the same measurement, wherein the 2D imaging plane 4 is approached from both out of plane directions. Further, calibrations are operator dependant since the operator has to indicate the position of features, of which he or she cannot see if it is in or out of the 2D imaging plane 4, for instance on a monitor. To judge whether the feature is in plane or not the operator needs to be experienced with US images, wherein each operator will have his or her own subjective threshold for deciding whether a feature is in or out of plane. Finally, the features to be indicated by the operator can extend the requested accuracy of 2mm. While a disciplined operator will try and pick the same point, relying on the operator's discipline carries a risk for as simple reasons as the duration of the calibration, which can take up to several hours.

The calibration method and system according to an embodiment of the present invention is illustrated in further detail in the following:

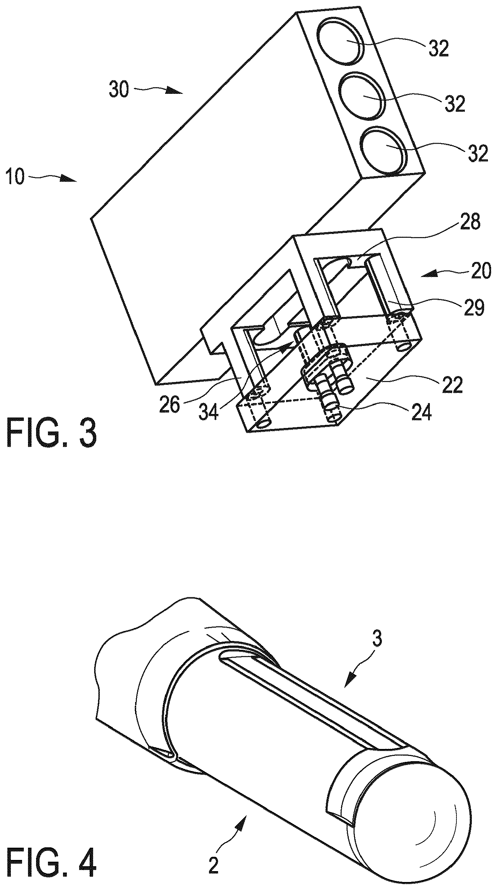

Fig. 3 schematically and exemplarily illustrates an example of a calibration block 10 according to an embodiment of the invention. Calibration block 10 comprises a mounting part 20 for mounting an ultrasound probe, such as ultrasound probe 2, to the calibration block 10 in a locked position. Mounting part 20 comprises a base plate 22, which integrates a fixation component 24, and a frame comprising a plurality of columns 26 linking the base plate 22 to a guidance pathways defining part 30.

Guidance pathways defining part 30 defines a plurality of coplanar guidance pathways 32, in this example guidance pathway defining part 30 defines three coplanar and parallel guidance pathways 32. Right through guidance pathways defining part 30, an acoustic slot 34 extends starting from the mounting part 20. In this example, acoustic slot 34 corresponds to hollow space, wherein in other examples, acoustic slot 34 can also comprise acoustically transparent material.

Fig. 4 schematically and exemplarily illustrates an ultrasound probe 2 to be received within the calibration block 10 or more precisely mounting part 20 thereof. Ultrasound probe 2 comprises an ultrasound or acoustical window 3 that is indicated, in this example, by two parallel stripes. The two parallel stripes of acoustical window 3 are aligned with the acoustic slot 34 as drawn in Fig. 3.

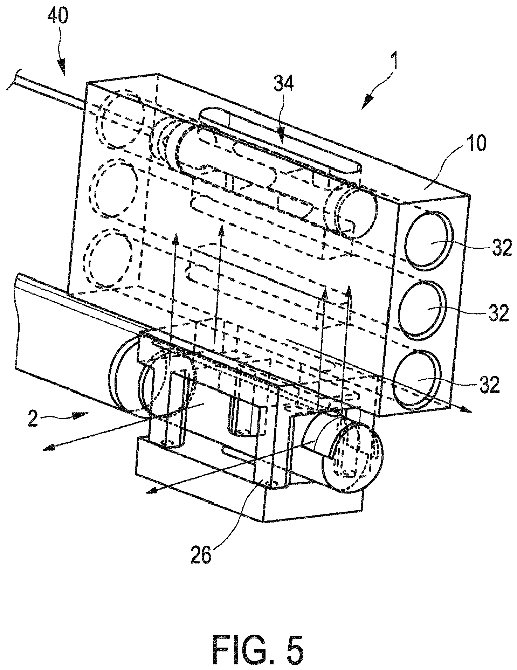

Fig. 5 schematically and exemplarily illustrates the mounting of ultrasound probe 2 to a calibration block 10 of a calibration system 1. Mounting part 20 locks the ultrasound probe 2 in six degrees of freedom, wherein the locked degrees of freedom are indicated by arrows in Fig. 5. Two degrees of freedom, represented as horizontal arrows directed to the left in Fig. 5, are locked by a tight-fit of the diameter of ultrasound probe 2 into a width of mounting part 20. More precisely, the width of two opposing columns 26 is 0.1mm, for instance, smaller than a diameter of ultrasound probe 2, so that the mounting part 20 locks ultrasound probe 2 therein by elastical restoring force. For example, calibration block 10 or mounting part 20 comprises an elastic material, such as plastic and more preferably PTFE. Returning to Fig. 3, inner sides 29 of columns 26 thus imply the restoring force onto ultrasound probe 2 in the mounted position.

Further, four degrees of freedom are locked by pulling the ultrasound probe against a contact surface component 28, which are oriented towards the guidance pathways defining part 30, by fixation component 24. Preferably, fixation component 24 comprises a screw, but also other means suitable for pulling ultrasound probe 2 against the surface component 28 are contemplated.

The locking is over constrained, which is in this example allowed by the small deviations of surfaces 28 and 29 and a small elastic deformation of the preferably elastic calibration block 10.

The only degree of freedom that is not locked by mounting part 20 is a longitudinal degree of freedom extending along the coplanar guidance pathways 32 or the ultrasound probe 2, respectively. This degree of freedom is chosen by an operator as desired.

The operation of calibration system 1 as schematically illustrated in Fig. 5 is now described in further detail by referring to certain components thereof.

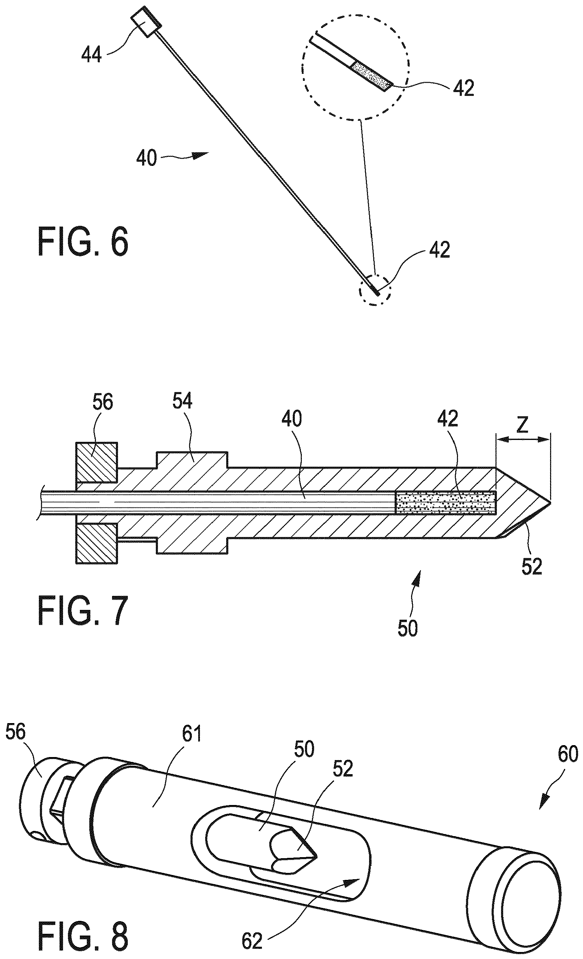

Fig. 6 schematically and exemplarily illustrates a calibration needle 40 comprising a tracked component or feature 42 at a distal portion thereof. On the opposite end, i.e. a proximal portion, a handle 44 is provided. Tracking feature 42 is for instance an electromechanically tracked sensor component as used in, for instance, tracking for brachytherapy.

The calibration needle 40 is in the example mounted in a trocar shaft 50 as illustrated in Fig. 7. Trocar shaft 50 comprises a trocar tip 52 having a feature, which is visible under ultrasound. At a certain distance z from the trocar tip 52, the tracked feature 42 of calibration needle 40 is arranged. Calibration needle 40 and trocar tip 52 are arranged coaxially, i.e. a central axis of trocar shaft 50. A relative distance between trocar tip 52 and tracked feature 42 is only in the said axial direction in this example, while there is no distance in a direction perpendicular thereto. The distance of trocar tip 52 with respect to tracked feature 42 is known by means of a measurement or by assembly and part accuracy. The trocar shaft 50 of Fig. 6 thus includes one feature that is clearly visible by ultrasound, i.e. trocar tip 52, and one feature of which a location is provided by the tracking system, i.e. tracked feature 42. These two features are used to generate the two point clouds with which the transformation is eventually calculated, as described below.

To bring the trocar shaft 50 in plane with the two-dimensional ultrasound images of ultrasound probe 2, calibration block 10 and a guidance part 60 as illustrated in Fig. 8 is used. Guidance part 60 receives trocar shaft 50 therein, wherein particularly trocar tip 52 is visible through an opening of window or aperture 62 extending through a guidance element 61 of guidance component 60. The acoustical window or aperture 62 extends perpendicular to the direction along the guidance pathways 32, wherein particularly the trocar tip 52 is visible through the acoustical aperture 62. In this example, guidance component 60 or guidance element 61 thereof comprises a substantially cylindrical shape, so that guidance component 60 can be rotated about its longitudinal axis while being received within the guidance pathway 32.

Outside of guidance element 61, a handle 56 of trocar shaft 50 is visible. Trocar shaft 50 is inserted into guiding element 61 of guidance component 60 up to a stop portion 54 thereof, which defines the position of trocar shaft 50 within guidance element 61.

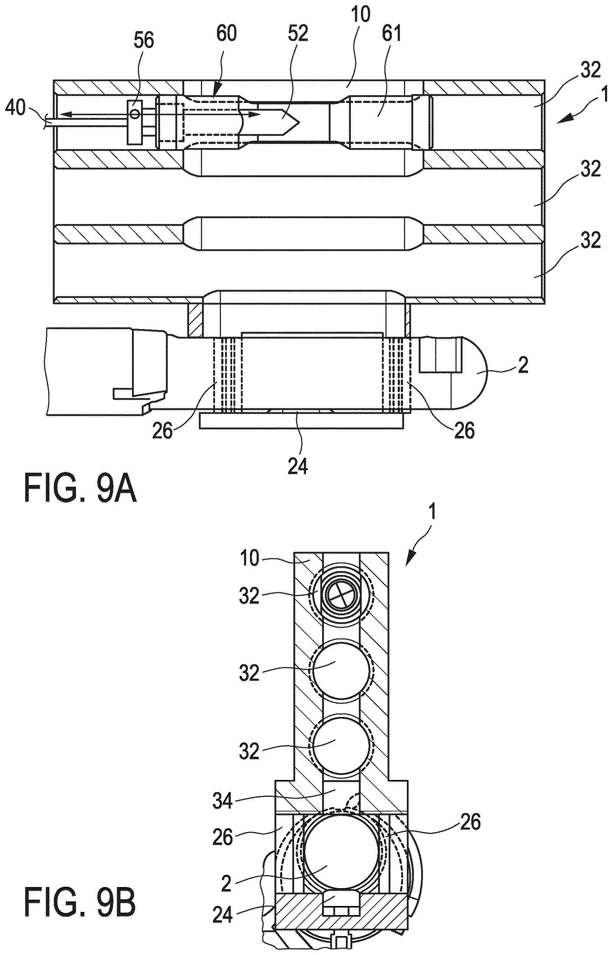

Trocar shaft 50 is thus mounted in the guidance component 60 that in return can slide through all pathways 32 in calibration block 10. Calibration block 10 is mounted onto the ultrasound probe 2 using preferentially external features of the ultrasound probe to align the guidance pathways 32 defined in guidance pathways defining part 30 with the two-dimensional ultrasound image plane of ultrasound probe 2 as described in two cross-sectional views in Fig. 9A and 9B.

Fig. 9A illustrates schematically and exemplarily a side view on calibration system 1, to which ultrasound probe 2 has been mounted. In Fig. 9A, a side view illustrating guidance component 60 within one of the coplanar guidance pathways 32 is illustrated. As indicated with an arrow, guidance component 60 and more importantly calibration needle 40 received therein and trocar shaft 50 are allowed to move back and forth within guidance pathway 32.

Likewise, Fig. 9B schematically and exemplarily illustrates a front view on calibration system 1. With an arrow in the highest of the coplanar guidance pathways 32 it is indicated that guidance component 62 is allowed to rotate about its axis within guidance pathway 32.

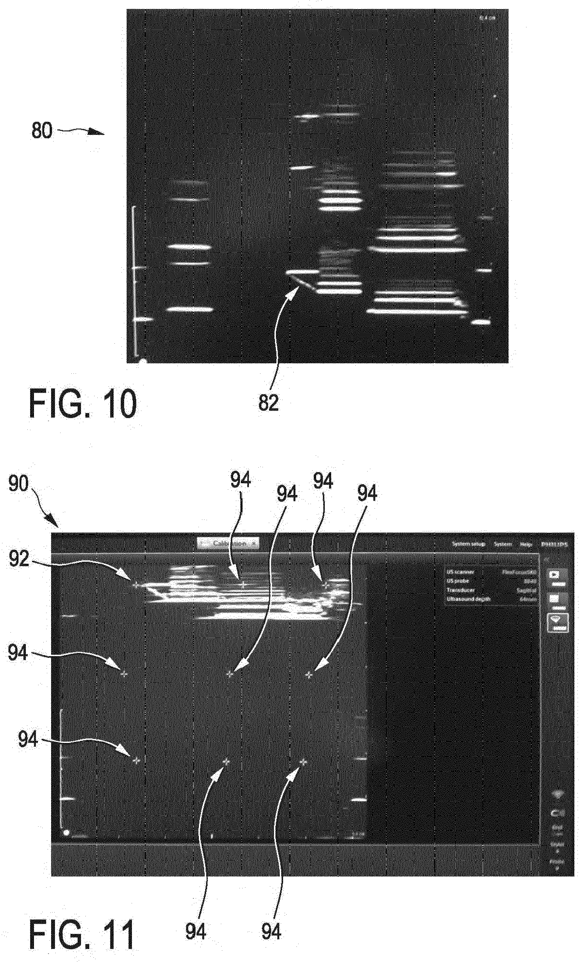

Fig. 10 schematically and exemplarily illustrates an ultrasound image acquired with ultrasound probe 2 using calibration system 1. Ultrasound image 80 corresponds to an ultrasound acquisition of the calibration block 10 mounted above the acoustical window of ultrasound probe 2. Therein, indicated with reference number 82, a reflection of guidance component 60 and most importantly of trocar tip 52 thereof is clearly visible.

Next, with reference to Fig. 11, the generating of a transformation between ultrasound image and tracking coordinate system is further illustrated. In addition, Fig. 11 illustrates an ultrasound image 90, which is displayed on a display apparatus, for instance, a monitor. Ultrasound image 90 corresponds to ultrasound image 80 in that trocar tip can clearly be identified at a position indicated with 92. Further, at each position trocar tip 52 can be identified, an operator marks the corresponding tip location, i.e. the feature visible in ultrasound, and at the same time, the position of the visible feature and the corresponding tracked location of the tracked feature are recorded. Guidance component 60 is advanced through all guidance pathways 32 and in all of the guidance pathways 32, multiple locations of the visible feature are identified and corresponding locations recorded.

In the example of Fig. 11, crosses indicated with 94 correspond to positions, at which the visible feature of guidance component 60 has already been identified. In summary, nine locations with arrow 94 and 92 have been identified, i.e. three per guidance pathway 32, which can then be used for creating the transformation matrix between both coordinate systems. In practice, an operator thus advances calibration component 60 through the various coplanar guidance pathways 32, picks the positions of the visual feature, e.g. trocar tip 52 positions, and simultaneously the tracking system reads out the position of tracking feature, e.g. tracking feature 42, mounted at a known position from the feature identifiable in the ultrasound image.

The calibration system 1 according to the invention thus pairs two coordinate systems, a coordinate system of visible points in the ultrasound image and a tracking technology coordinate system, such as an electromagnetical tracking coordinate system. A transformation matrix is yielded used to project images obtained by ultrasound probe 2 into the tracking system coordinates. In this tracking coordinate system, the position of the ultrasound images is combined with the positions of tracked devices, for instance catheters, stylets and the like.

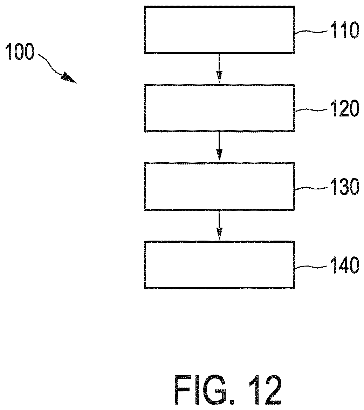

Finally, with respect to Fig. 12 a calibration method 100 for determining transformation between an ultrasound image and a tracking coordinate system using a calibration system 1 is described.

Calibration method 100 comprises a step 110 of mounting an ultrasound probe to the mounting part 20 of the calibration block 10 as described above.

Next, in a step 120, a guidance component 60 is inserted into a guidance pathway 32, wherein the guidance component 60 is configured to guide a feature visible in an ultrasound image and a tracked feature along the guidance pathway 32. As exemplified, the feature visible in an ultrasound image can for instance be the trocar tip 52 and the tracked feature can, for instance, be a tracked feature 42 arranged at a distal tip of a calibration needle 40.

Next, in a step 130, multiple corresponding positions of the feature visible in the ultrasound image and the tracked feature are recorded at the same position of the guidance component 60 within the guidance pathway 32. Subsequently, a plurality of positions is taken or recorded, wherein the guidance component 60 eventually travels all of the available guidance pathways 32.

Finally, in a step 140, a transformation between the ultrasound image and the tracking coordinate system is determined based on the recorded multiple corresponding positions. In an example, the transformation comprises a transformation matrix determined by any suitable method known in the art, for instance comprising a singular value decomposition method.

Calibration method 100 can be used for all ultrasound probes 2 onto which an external block such as calibration block 10 can be mounted which aligns the pathway of a device visible in ultrasound and visible by the tracking technology.

The invention and calibration method 100 preferentially are used with EM tracking as the tracking technology. However, the teaching of the invention can also be used for other combinations, in which a US imaging system, such as imaging probe 2 is used with a tool tracking system, for instance optical tracking. Advantageously, calibration block 10 of calibration system 1 according to the invention ensures the needle tip 42 being in plane with the ultrasound image.

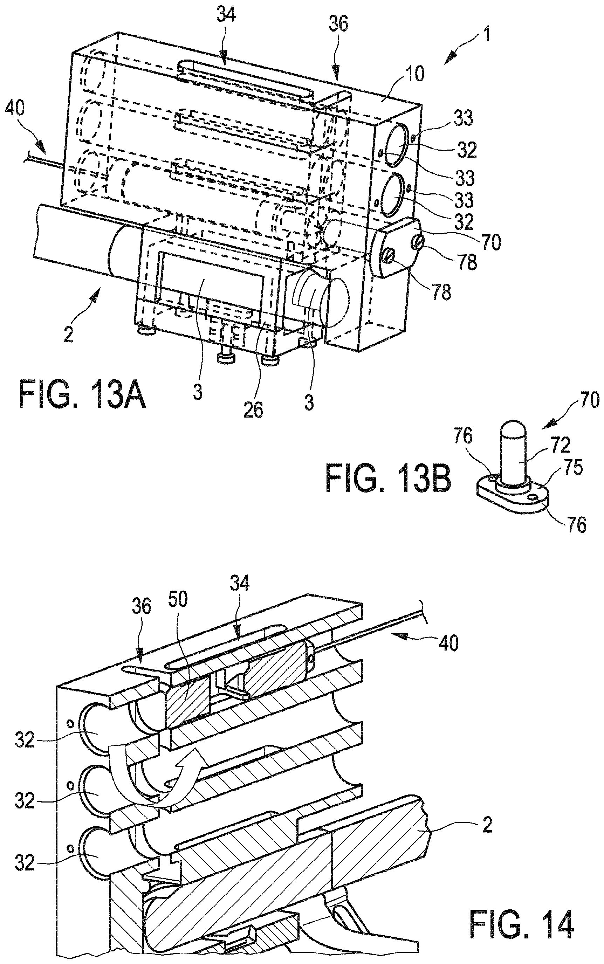

Fig. 13A and 13B schematically and exemplarily illustrate a further example of calibration system 1, in which additionally an end stop component 70 is mounted within one of the guidance pathways 32. An example of the isolated end stop component 70 is schematically illustrated in Fig. 13B. End stop component 70 comprises a cylindrical portion 72, which is configured to fit inside the guidance pathway 32. To an end thereof, plate 74 is attached, which is to remain outside the guidance pathway 32. Plate 74 is used for inserting and removing end stop component 70 into and from the guidance pathways. Further, plate 74 comprises in this example two attachment holes 76, into which fixation means 78, such as screws, can be inserted. Fixation means 78 match with corresponding fixation means 33 adjacent to an opening of guidance pathway 32 in order to securely attach end stop component 70 to calibration block 10. It should be noted that the screws are of course only one example for a suitable fixation means 78 and also other examples are of course possible. Advantageously, when the probe with mounted calibration block 10 is in a vertical position, the guidance component 60 can rest on the end stop component 70, while the user can manipulate or operate, for instance, a software program. Thus, a position of the guidance component 60 can be maintained reliably. By using end stop components 70 of different lengths, the location of the secured position of guidance component 60 can be varied in a reproducible manner.

Further, as can be seen in the example of Fig. 13A and as further detailed in Fig. 14, calibration block 10 comprises a first acoustic slot 34 and a second acoustic slot 36. Correspondingly, ultrasound probe 2 comprises two acoustical windows 3 aligned with each of the first acoustic slot 34 and the second acoustic slot 36, respectively. More specifically, ultrasound probe 2 comprises a transverse transducer, which is aligned with acoustic slot 36 in addition to the sagittal transducer, which is aligned with acoustic slot 34 in this example.

Fig. 14 schematically and exemplarily illustrates a perspective sectional view through calibration block 10 of Fig. 13A. In this example, second acoustic slot 36 being arranged in a transverse direction can be clearly seen. While first acoustic slot 34 and second acoustic slot 36 are separated by a portion of guidance block 10 in this example, they can also be connected in other examples. The specific position of each of the first and second acoustic slot 34, 36 is rather exemplarily, as long as both slots substantially extend in respective directions corresponding to the imaging planes of the ultrasound probe 2. It should be noted that in the example using the additional transverse transducer, the end stop component 70 as illustrated in Fig. 13 is required to guarantee that the guidance component 60 and more specifically the tip thereof is arranged in the transverse imaging plane.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit, component or device may fulfill the functions of several items recited in the claims. The mere fact that certain measures are recited in mutually different dependent claims does not indicate that a combination of these measures cannot be used to advantage.

## Claims

1. A calibration block for determining a transformation between an ultrasound (US) image and a tracking coordinate system, the calibration block (10) comprising
a mounting part (20) for mounting an ultrasound probe (2) to the calibration block (10) in a locked position, and
a guidance pathways defining part (30) for defining a plurality of coplanar guidance pathways (32),
wherein the calibration block (10) is configured to receive the ultrasound probe (2) in the locked position so that the plurality of guidance pathways (32) is aligned with an imaging plane of the ultrasound probe (2).

2. The calibration block as defined in claim 1, further comprising an acoustic slot (34) extending from the mounting part (20) through the guidance pathways defining part (30), wherein the acoustic slot (34) intersects the guidance pathways (32).

3. The calibration block as defined in claim 1, wherein the calibration block (10) comprises a fixation component (24), in particular a screw, for securing the ultrasound probe (2) in the locked position.

4. A calibration system for determining a transformation between an ultrasound (US) image and a tracking coordinate system, wherein the calibration system (1) comprises:
a calibration block (10) as defined in claim 1, and
a guidance component (60) for being inserted into at least one of the guidance pathways (32), wherein the guidance component (60) is configured to guide a feature (52) visible in an ultrasound image and a tracked feature (42) along the guidance pathway (32).

5. The calibration system as defined in claim 4, wherein the calibration system (1) further comprises a processing unit, wherein the processing unit is configured to:
record multiple corresponding positions (92, 94) of the feature (52) visible in the ultrasound image and the tracked feature (42) along the guidance component (60), and to
determine a transformation between the ultrasound image and the tracking coordinate system based on the recorded multiple corresponding positions (92, 94).

6. The calibration system (1) as defined in claim 4, wherein a relative position of the feature (52) visible in the ultrasound image and the tracked feature (42) is fixed.

7. The calibration system (1) as defined in claim 4, wherein the system further comprises a calibration needle (40) comprising a tracked sensor component (42), wherein the guidance component (60) is configured to receive the calibration needle (40) therein.

8. The calibration system (1) as defined in claim 4, wherein the guidance component (60) comprises a guidance element (61) and a trocar shaft (50) to be mounted within the guidance element (61), wherein a tip of the trocar shaft (52) corresponds to the feature visible in an ultrasound image (90).

9. The calibration system (1) as defined in claim 8, wherein the guidance element (61) comprises an acoustical aperture (62) perpendicular to the direction along the guidance pathway, wherein the tip of the trocar shaft (52) is acoustically visible through the acoustical aperture (62).

10. The calibration system (1) as defined in claim 8, wherein the trocar shaft (50) comprises a needle channel for receiving a calibration needle (40) therein, wherein an end of the needle channel is located at a determinable position with respect to the tip of the trocar shaft (52).

11. The calibration system (1) as defined in claim 4, wherein both the guidance pathways (32) and the guidance component (60) have a cylindrical shape so that the guidance component (60) is rotatable when the guidance component (60) is received within one of the guidance pathways (32).

12. The calibration system (1) as defined in claim 4, the system (1) further comprising an end stop component (70), the end stop component (70) being configured to be inserted into one of the guidance pathways (32) for defining a limit stop of the guidance component (60) along the guidance pathway (32).

13. A guidance component for use with a calibration block for determining a transformation between an ultrasound (US) image and a tracking coordinate system according to claim 1, the calibration block (10) comprising a guidance pathways defining part (30) for defining a plurality of coplanar guidance pathways (32) for being inserted into at least one of the guidance pathways (32), the guidance component (60) being configured to guide a feature (52) visible in an ultrasound image and a tracked feature (42) along the guidance pathway (32).

14. A calibration method for determining a transformation between an ultrasound (US) image and a tracking coordinate system using a calibration system (1) as defined in claim 7, wherein the calibration method (100) comprises:
mounting an ultrasound probe (2) to the mounting part (20) of the calibration block (10)
inserting a guidance component (60) into a guidance pathway (32), wherein the guidance component (60) is configured to guide a feature (52) visible in an ultrasound image (90) and a tracked feature (42) along the guidance pathway,
recording multiple corresponding positions (92, 94) of the feature visible in the ultrasound image (90) and the tracked feature (42) at the same position of the guidance component (60), and
determining a transformation between the ultrasound image (90) and the tracking coordinate system based on the recorded multiple corresponding positions.

15. A computer program for determining a transformation between an ultrasound (US) image and a tracking coordinate system, the computer program comprising program code means for causing a processing unit to carry out the calibration method (100) as defined in claim 14, when the computer program is run on a processing unit of a calibration system (1) as defined in claim 7.
